# EUROPEAN PATENT SPECIFICATION

(11) **EP 1 317 256 B1**
(45) Date of publication and mention of the grant of the patent: **29.10.2008**
(21) Application number: 01968045.3
(22) Date of filing: 07.09.2001
(51) Int. Cl.: A61K 9/48

(54) **PHARMACEUTICAL COMPOSITION HAVING MODIFIED CARRIER**
PHARMAZEUTISCHE ZUSAMMENSETZUNG MIT MODIFIZIERTEM TRÄGER
COMPOSITION PHARMACEUTIQUE DOTEE D'UN SUPPORT MODIFIE

(30) Priority: 12.09.2000 US 231767 P
(43) Date of publication of application: 11.06.2003
(73) Proprietor: Pharmacia & Upjohn Company LLC, Kalamazoo, MI 49001 (US)
(72) Inventor: TEAGARDEN, Dirk, L., Kalamazoo, MI 49002 (US); BRITTEN, Nancy, J., Portage, MI 49024 (US); BROWN, Scott, A., Galesburg, MI 49053 (US); CAPUTO, James, F., Portage, MI 49002 (US); EATON, Leslie, C., Schoolcraft, MI 49087 (US); HENDL, Ondrej, Portage, MI 49024 (US); HUDA, Syed, F., Karachi (PK); KING, Harry, M., Portage, MI 49024-6617 (US); MACHKOVECH, Susan, M., Mattawan, MI 49071 (US); SCHAPAUGH, Randal, Lee, Battle Creek, MI 49014 (US); SPEAKER, Stanley, M., Portage, MI 49002 (US); STEELE, Jean, M., Portage, MI 49024 (US); SU, Ching-Chiang, Kalamazoo, MI 49009 (US); URBAN, Terry, R., Portage, MI 49024 (US); WALDRON, Niki, A., Kalamazoo, MI 49009 (US); WHITMIRE, Monica, L., Kalamazoo, MI 49009 (US)
(74) Representative: Rutt, Jason Edward
(86) International application number: PCT/US2001/026013
(87) International publication number: WO 2002/022107

(56) References cited:
- US-A- 5 721 359
- US-A- 5 736 151
- US-A- 6 074 657

## Description

### Background of the Invention

### 1. Field of the Invention

The present invention relates to pharmaceutical compositions. More specifically, it relates to the use of modified pharmaceutically acceptable liquid carriers that provide the composition with predictable sustained-release properties.

### 2. Background of the Invention

In the pharmaceutical arts, drug delivery is an element as significant as drug activity. Many drugs or bioactive agents with apparent *in vitro* activity fail at the clinical level due to the inability to prepare, store, or deliver the bioactive agent to the site of action in effective concentrations over a sufficient period of time.

A vehicle for the stable storage and effective delivery profile of a bioactive agent is of great utility. Those skilled in the art will understand that storage stability and effective delivery profile are, to some extent, specific to bioactive agents, the condition for which the bioactive agent is administered, and the presenting condition of the subject.

Sustained-release or oil-based preparations are considered in WO97/49402 (Vlaminck); WO94/00105 (Sabater); US 4,297,353 (Hawkins); US 5,019,395 (Mahjour); US 5,739,159 (Wolf); US 5,162,057 (Akiyama); WO96/20698 (Levy) the teachings of which are incorporated herein by reference. Also incorporated by reference is WO98/41207 (Brown) addressing subcutaneous (S.C.) administration of antibiotic into the ear of an animal.

US 5,721,359 discloses the molecule crystalline ceftiofur free acid (CCFA), which is a cephalosporin antibiotic intended for use in mammals, and in particular food animals (e.g., cattle, sheep, goats and swine). The patent suggests that oil suspensions of CCFA can be produced for administration to food animals where the oils are vegetable oils. The oils as disclosed in the patent are intended to be used in their natural form. An advantage touted by this molecule over other antibiotics, particularly those in the ceftiofur family is the ability for CCFA to yield a sustained-release pharmaceutical composition. It has now been discovered that the sustained-release profile is not readily predictable and reproducible in immediate post-production product that uses natural vegetable oils.

Despite the above teachings, there still exists a need in the art for pharmaceutical compositions that can be administered on a sustained-release basis and wherein the release performance is predictable and reproducible immediately after manufacture of the product.

### Brief Summary of the Invention

In accordance with the present invention a novel pharmaceutical composition that can be administered on a sustained-release basis and wherein the release performance is predictable immediately after manufacture of the product is provided. More specifically, the predictable performance is obtained by using a modified liquid carrier.

One embodiment of the invention provides a pharmaceutical composition comprising:
(a) one or more bioactive agents; and
(b) a modified liquid carrier;
wherein immediately after manufacture of the composition, said composition can be administered to a host such that the one or more bioactive agents are released to the host on a predictable sustained-release basis.

In preferred embodiments, component (a) comprises CCFA and component (b) must include at least one of the following: (1) a modified unsaturated oil; (2) a modified saturated oil; (3) a modified non-oxidizing vehicle; or (4) a modified non-oil. In particularly preferred embodiments component (b) is a mixture of a modified unsaturated oil with a natural fully saturated oil, and even more preferably a mixture of modified cottonseed oil with saturated coconut oil.

Another embodiment of the present invention provides a method for producing a pharmaceutical composition comprising the step of modifying a liquid carrier and combining said modified liquid carrier with a bioactive substance. According to this method the liquid carrier is modified by the use of chemical, physical or mechanical means to produce a carrier that has a higher level of oxidation products as compared to its original, or non-modified form. Particularly preferred embodiments comprise the use of a combination of heat and gamma radiation. In addition, the modification step of this process may occur either prior to, after or both prior to and after the combining step.

A more specific aspect of this method comprises the steps of:
(a) heating natural cottonseed oil to increase its oxidation products and yield a modified cottonseed oil;
(b) combining said modified cottonseed oil with saturated coconut oil or saturated coconut oil products to yield a carrier vehicle; and
(c) adding crystalline ceftiofur free acid to said carrier vehicle; and, optionally, thereafter;
(d) heating said pharmaceutical composition;
(e) cooling said composition;
(f) filling one or more vials with said composition; and
(g) exposing said one or more vials to gamma radiation.

A further embodiment of the present invention provides the composition of the present invention for use in medical treatment.

An additional embodiment of the present invention provides the use of the inventive composition to prepare a medicament for treating or preventing a disease in a mammal.

A final embodiment of the present invention provides a method of treating or preventing a disease comprising administering to a mammal in need of such treatment an effective amount of the inventive composition. A preferred aspect of this invention is to treat bacterial infections in food animals or companion animals with an inventive CCFA composition.

An object of the present invention is to provide a novel sustained-release composition.

Still another object of the present invention is to provide a method for producing a novel sustained-release composition.

A further object of the present invention is to provide a method for treating a disease or condition in a mammal.

These, and other objects, will readily be apparent to those skilled in the art as reference is made to the drawings and detailed description of the preferred embodiment.

### Brief Description of the Drawings

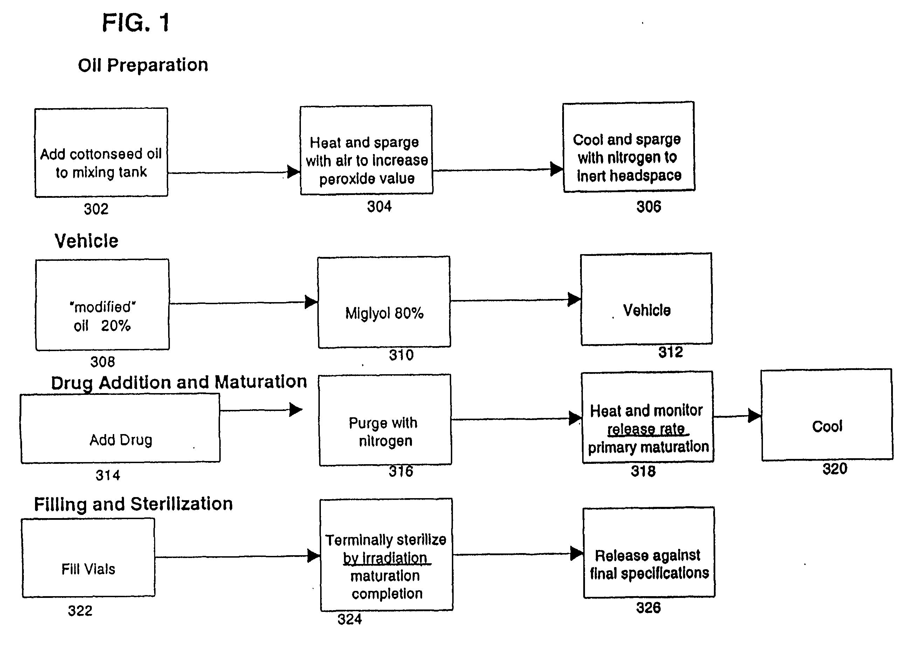

Fig. 1 is a diagram of a method that can be used to produce the inventive compositions.

### Detailed Description of the Invention

In describing the preferred embodiment, certain terminology will be utilized for the sake of clarity. Such terminology is intended to encompass the recited embodiment, as well as all technical equivalents which operate in a similar manner for a similar purpose to achieve a similar result.

### 1. Terminology Definitions

This invention will be better understood with reference to the following definitions:

"Bioactive substances" shall be broadly understood to mean pharmaceuticals, immunogenic and immunomodulator compositions (including adjuvants), vectors such as liposomes and live vectors such as plasmids, viruses, prions, spores, nutritional supplements and bacteria and mixtures thereof.

These include, but are not limited to nutritional supplements, anti-infectives (*e.g.*, antibiotics, antifungals, anti-virals), antineoplastics (*e.g.*, anticancer agents, such as cis-platinum compounds), immunomodulators (*e.g.*, antihistamines, immunoenhancers and immunosupressors), laxatives, vitamins, decongestants, gastrointestinal sedatives, antacids, anti-inflammatory substances, anti-manics, vasodilators (coronary, cerebral and peripheral), psychotropics, narcotics, stimulants, anti-diarrheal preparations, anti-anginal drugs, analgesics, anti-pyretics, hypnotics, sedatives, anti-emetics, growth promoters, anti-nauseants, anti-convulsants, neuromuscular drugs, hyper and hypo glycemic agents, thyroid and anti-thyroid preparations, diuretics, cytotoxic compounds, anti-spasmodics, anti-arthritics, uterine relaxants, anti-obesity drugs, anthelmintics, hormones, vaccines, mineral and nutritional additives, CNS agents not disclosed in this listing and any mixtures thereof. Specifically preferred bioactive agents include, but are not limited to, ceftiofur, including crystalline ceftiofur free acid (CCFA), platinum compounds (*e.g.*, *cis*-platinum), ibuprofen, piroxicam, 1-[2-(4-fluorobenzoyl)aminoethyl]-4-(7-methoxynaphthyl) piperazine hydrochloride (FAMP), camptothecin, paclitaxel, flucytosine, cyclooxygenase-II inhibitors (e.g., coxibs and chromenes) and quinine.

"Sustained-delivery or Sustained-release" as used in relation to bioactive substances shall mean continued release or distribution of the bioactive substance such that the amount of bioactive remains in the patient's blood levels at a concentration of greater than a certain value (that value being one that produces therapeutically effective blood levels of active substance) over an extended period of time. The effective sustained-release blood levels desired would, of course, differ depending on the bioactive substance, the disease being treated, the patient, and the like, is considered to be known to the skilled artisan and can be determined by routine experimentation. More specifically, a sustained-delivery vehicle differs from an immediate- delivery vehicle in that the immediate-delivery vehicle releases its bioactive material at faster rate then the sustained-delivery vehicle, potentially requiring more administrations of bioactive per treatment regimen. For example, if the bioactive substance is ceftiofur crystalline free acid (CCFA), the desired level of ceftiofur metabolites in the patient's blood plasma is noted to be maintained at or above about 0.2 µg/ml. In one embodiment of the invention, a single dose of sustaining-vehicle CCFA maintains a ceftiofur metabolite level in the blood plasma of at or above about 0.2 µg/ml for at least three and preferably at least about four and more preferably at least about five days post-administration (sustained delivery of CCFA). Comparisons as to the degree of sustained delivery are made with equivalent bioactive agents. That is, sodium salts to sodium salts and free bases to free bases. Sustained-delivery as used in this document is to be specifically reconciled with the regulatory definition for the same term that requires that the concentration versus time profile have three distinct phases (i.e., an increasing concentration phase, a plateau phase and a concentration depletion phase). While the term sustained-delivery as used in this document may encompass the above regulatory definition it is not intended to be limited to it as compositions which are sustained delivery as defined herein need not possess the three distinct phases (e.g., the composition may have an increasing concentration phase and an extended concentration depletion phase).

"Liquid carriers" include triglyceride fats and oils, including those derived from vegetable, animal, and marine sources. In practice it is preferred that-the liquid carrier is non-aqueous, although the use of aqueous carriers is contemplated. The liquid carrier may be fully saturated, partially or fully unsaturated and may be deemed an "oil" (which may be naturally occurring or synthetic) or a "non-oil". Examples of liquid carriers which are partially or fully unsaturated hydrocarbons include, but are not limited to naturally occurring oils such as castor oil, safflower oil, cottonseed oil, corn oil, olive oil, cod liver oil, almond oil, avocado oil, palm oil, sesame oil, peanut oil, and soybean oil. By way of example cottonseed oil is available in a preparation of 70% unsaturated fatty acids (Sigma, St. Louis, MO). Examples of fully saturated liquid carriers include, but are not limited to, esters of medium to large chain fatty acids (e.g., fatty acid triglycerides with a chain length of about C₆ to about C₂₄). Mixtures of fatty acids are split from the natural oil (e.g., coconut oil, palm kernel oil, babassu oil, etc.) and are refined. In some embodiments, about C₈ to about C₁₂ fatty acid medium chain triglycerides (MCT) are useful. These saturated vehicles are comprised of capric acid (about 20 to about 45%) and caprylic acid (about 45 to about 80%). Fully saturated liquid carriers include, but are not limited to, saturated coconut oil (which typically includes a mixture of lauric, myristic, palmitic, capric and caprylic acids), including those sold under the MIGLYOL trademark from Huls and bearing trade designations 810, 812, 829 and 840. Also noted are the NeoBeeRTM products sold by Drew Chemicals. Isopropyl myristate is another example of a non-oxidizing vehicle of the current invention. Examples of synthetic oils include triglycerides, or propylene glycol di-esters of saturated or unsaturated fatty acids having from 6 to 24 carbon atoms. Such carboxylic acids are meant to comprise those carboxylic acids having from 6 to 24 carbon atoms such as, for example hexanoic acid, octanoic (caprylic), nonanoic (pelargonic), decanoic (capric), undecanoic, lauric, tridecanoic, tetradecanoic (myristic), pentadecanoic, hexadecanoic (palmitic), heptadecanoic, ocadecanoic (stearic), nonadecanoic, hexadecanoic (palmitic), heptadecanoic, ocatdecanoic (stearic) nonadecanoic, eicosanoic, heneicosanoic, docosanoic and lignoceric acid. Examples of unsaturated carboxylic acids include oleic, linoleic, linolenic acid and the like. It is understood that the tri-glyceride vehicle may include the mono-, di-, or triglyceryl ester of the fatty acids or mixed glycerides and/or propylene glycol di-esters wherein at least one molecule of glycerol has been esterified with fatty acids of varying carbon atom length. Some non-limiting examples of "non-oils" include polyethylene glycol (PEG) and aqueous based vehicles.

"Modified" and "modification" as to the vehicles of this invention and as used in the claims shall be understood to define a vehicle which, through physical, chemical or mechanical means, has been altered as compared to its natural (or "non-modified" in the case of synthetic liquid carriers) form such that the modified vehicle has an increased level of oxidation products. Modification can be accomplished by heat modification, irradiation and/or exposure to energy sources (e.g., light, ultraviolet, infrared, gamma, X-ray or microwave radiation), addition of catalysts (i.e., t-butyl peroxide), the incorporation of specific triglycerides and their hydroperoxides, incorporation of polymeric species, incorporation of crosslinkers or of polymerization causing agents, oxidation regimens and combinations of these methods. These steps can be taken before or after addition of the drug to the vehicle, or both before and after addition of drug to the vehicle. In accordance with preferred embodiments of the present invention, the modification takes place in connection with the pharmaceutically acceptable liquid carriers which are either partially or fully unsaturated, although it is specifically contemplated that modification of the fully saturated liquid carriers or non-oils is also possible.

"Substantially peroxidized unsaturated oil vehicle" shall refer to a modified liquid carrier having a peroxide value of between about 0.1 and about 600, and in some embodiments about 10, about 20, about 40, or about 80 or any value in between. As used herein, peroxide values are expressed as milliequivalents (mEq) of peroxide per 1000 grams of oil sample. Peroxide value is conveniently measured by American Oil Chemists' Society (AOCS) (Official Method Cd 8-53)(Official Monographs, Soybean Oil, page 1434) manual titration, the teachings of which are incorporated herein by reference.

### 2. The Invention

The present invention comprises a composition comprising:
(a) one or more bioactive agents; and
(b) a modified liquid carrier; -
wherein immediately after manufacture of the composition, said composition can be administered to a host such that the one or more bioactive agents is released to the host on a sustained basis.

It is a substantial advantage to identify a dosage form and method of preparation of a dosage form that provides sustained-release capability immediately upon production and maintains that release profile during a substantial storage period. In the present invention, a combination of preparatory steps and vehicle compositions are defined which yield sustained-release formulations upon processing. This is obtained by the use of a modified liquid carrier, which can be in the form of a modified unsaturated oil, a modified saturated oil, a modified non-oxidizing vehicle, a modified non-oil or any mixture thereof in combination with the bioactive agent. In addition to the modified component, the liquid carrier can also optionally include a non-modified unsaturated oil, a non-modified saturated oil, a non-modified non-oxidizing vehicle, a non-modified non-oil or any mixture thereof. In all embodiments, a key feature is that a portion of the carrier vehicle has been modified either before, after or both before and after it has been combined with the bioactive agent.

The bioactive agents for use are as defined above. A preferred bioactive agent is crystalline ceftiofur free acid (CCFA) which is useful as an antibiotic drug compound in pharmaceutical dosage forms for treating valuable mammalian animals and humans suffering from bacterial infections. In particular embodiments, sustained-release ceftiofur free acid is useful as a veterinary antibiotic drug to treat animals such as cattle, swine, horses, sheep, goats, dogs, poultry and cats. Such treatment fights the effects of bacterial infections caused by susceptible organisms, such as *Pasteurella haemolytica (Mannheimia Spp.), Pasteurella multocida, Salmonella typhimurium, Salmonella choleraesuis, Actinobacillus pleuropneumoniae, Streptococcus suis, Streptococcus equi (zooepidemicus),* and other *Streptococcus* bacteria, *Haemophilus somnus, Escherichia coli, Staphylococcus aureus* and the like, as well as applicable anaerobic infections, such as *Fusobacterium necrophorum.* These types of infections are commonly associated with diseases in animals, such as bovine respiratory disease and swine respiratory disease.

In specific embodiments, the following are used as the carrier vehicles of the instant invention:
(1) a mixture of a modified unsaturated oil with either a non-modified saturated oil or a non-modified non-oxidizing vehicle;
(2) a modified unsaturated oil;
(3) a mixture of a non-modified unsaturated oil and either a modified saturated oil or a modified non-oxidizing vehicle;
(4) a modified saturated oil or a modified non-oxidizing vehicle; or
(5) a modified non-oil.

Other combinations which include as at least one component a modified vehicle can be envisioned by one skilled in the art and are expressly covered as being within the scope of the instant invention.

A preferred embodiment of the present invention is where the delivery vehicle is the combination of a modified unsaturated oil with a fully saturated oil or non-oxidizing vehicle. In even more preferred embodiments, the modified unsaturated oil is a substantially peroxidized unsaturated oil vehicle. For this embodiment, the ratio of modified unsaturated oil to saturated, non-oxidizing oil is from about from about 0.01:99.99 to about 90:10 (v/v), the total amount of each being 100 percent, with particular reference to the range from about 10:90 to about 25:75 (v/v), and most particularly from about 10:90 to about 20:80 (v/v).

An example of this is if the modified unsaturated oil comprises modified cottonseed oil and the non-modified saturated oil or non-modified non-oxidizing vehicle comprises saturated coconut oil or a saturated coconut oil product (for example MIGLYOL 812). So called "induced" cottonseed oil which has a higher level of oxidation products as a result of natural cottonseed oil having been heated in the presence of oxygen is specifically contemplated as being a type of modified cottonseed oil. When the bioactive agent is CCFA, it is preferably combined with this example vehicle such that the concentration of the CCFA in the composition ranges between about 50 mg/ml to about 250 mg/ml and more preferably between about 100 mg/ml to about 200 mg/ml.

Fig. 1 presents a useful processing scheme for producing a sustained-release product of this embodiment. Natural (non-modified) cottonseed oil is added to a mixing tank (302) which is then heated and sparged with air to increase the peroxide value (304). The cottonseed oil is then cooled and sparged with nitrogen (306). The cottonseed oil at this point is deemed modified cottonseed oil. The vehicle (312) is then prepared by mixing 20 parts by volume of modified cottonseed oil (308) with 80 parts by volume of a saturated coconut oil or saturated coconut oil product, for example Miglyol 812. (310). Drug (bioactive substance, for example CCFA) is added to the vehicle (314) and the mixture is purged with nitrogen (316). The purged mixture is heated and the release rate of the drug is monitored using an in process assay procedure to determine when the desired release rate is achieved. At this point the heating is terminated (318) and the mixture is cooled (320), filled into vials (322) and terminally sterilized by gamma irradiation (324) and released against final specifications (326).

Revisiting the process of Fig. 1, it is contemplated that sustained-release formulations of other embodiments can be achieved by alternate routes within the disclosed process. For example, in one such process, drug is added to a non-modified unsaturated oil and directly subjected to terminal irradiation to modify the unsaturated oil and produce a sustained-release vehicle. In another, the process is terminated after fill and without terminal sterilization. In an embodiment consisting of a modified non-oil such as PEG 400, the drug/PEG-400 mixture is purged with nitrogen, heated, cooled and filled. It is an important aspect of the invention that not all processing steps are required to result in a sustained-release preparation in every protocol. However, in accordance with the present invention some type of chemical, physical, or mechanical modification or any combination of the above is required.

In addition to the instant inventive vehicle of the instant invention, the compositions of this invention can be employed in admixture with conventional excipients, i.e., pharmaceutically acceptable organic or inorganic carrier substances suitable for parenteral, enteral (e.g., oral or inhalation) or topical application which do not deleteriously react with the active compositions. Suitable pharmaceutically acceptable carriers include but are not limited to water, salt solutions, alcohols, gum arabic, vegetable oils, benzyl alcohols, polyethylene glycols, gelatine, carbohydrates such as lactose, amylose or starch, magnesium stearate, talc, silicic acid, viscous paraffin, perfume oil, fatty acid esters, hydroxy methylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compositions. They can also be combined where desired with other active agents, e.g., vitamins. In specific embodiments, the liquid carrier may additionally contain a thickening agent, for example, beeswax, hard paraffin or cetyl alcohol. Also noted as optional additives are benzyl alcohols, polyethylene glycols, viscous paraffin, perfume oil, and fatty acid esters.

The inventive compositions are useful for human and veterinary medicine. More specifically, the compositions of the present invention can be used to treat humans, food animals or companion animals. This includes, but is not limited to the following: food animals such as cattle, swine, sheep, goats and deer; companion animals such as horses, cats and dogs; poultry; or humans. The amount of inventive composition to be administered is that which will deliver the bioactive agent in an amount and for a duration to provide a therapeutic benefit necessary to treat or prevent a disease without causing toxicity problems to the patient. The specific amounts to be selected are deemed to be within the skill of the artisan. For example, when CCFA is selected as the bioactive agent, it is administered in unit dosage form for intramuscular or subcutaneous administration comprising about 0.5 to about 10.0 mg CCFA/kg body weight of patient with preferred ranges of about 4.4 - 6.6 mg/kg for cattle, and 5.0-7.5 mg/kg for swine. To the extent necessary for completion the dosages as described in US 5,721,359 and US 6,074,657 are expressly incorporated by reference.

Administration of the composition is contemplated to include chronic, acute or intermittent regimens, and any mode where liquid administration is feasible may be selected. The compositions of the present invention can be administered parenterally (for example, subcutaneous, intramammary, intravenous, intraperitoneal or intramuscular injection), topically (including but not limited to surface treatment, transdermal application, and nasal application), intravaginally, orally, or rectally.

For oral therapeutic administration, the composition may be administered in the form of capsules, elixirs, suspensions, syrups, and the like. Such compositions and preparations should, typically, contain at least 0.1% of active compound. The percentage of the compositions and preparations may, of course, be varied and may conveniently be between about 2 to about 60% of the weight of a given unit dosage form. The amount of active compound in such therapeutically useful compositions is such that an effective dosage level will be obtained. A syrup or elixir may contain the active compound, sucrose or fructose as a sweetening agent, methyl and propylparabens as preservatives, a dye and flavoring such as cherry or orange flavor. Of course, any material used in preparing any unit dosage form should be pharmaceutically acceptable and substantially non-toxic in the amounts employed. In addition, the active composition may be incorporated into sustained-release preparations and devices such as the osmotic release type devices developed by the Alza Corporation under the OROS trademark.

For parenteral application, the compositions can be administered intravenously or intraperitoneally, by infusion or injection. In one embodiment where CCFA is the bioactive agent, subcutaneous ear injection in accordance with US 6,074,657 is an appropriate mode of administration. Intramuscular, intramammary and general subcutaneous administration is also specifically contemplated.

For topical administration, the composition may be applied in the form of drops (for example to treat diseases or infections of the eye), or for skin application in the form of spreadable pastes, gels, ointments, soaps, and the like. The resultant liquid compositions can additionally be applied from absorbent pads or suppositories, used to impregnate bandages and other dressings, or sprayed onto the affected area using pump-type or aerosol sprayers.

It will be appreciated that the actual preferred amounts of active compositions in a specific case will vary according to the specific compositions being utilized, the particular compositions formulated, the mode of application, and the particular situs and organism being treated. Dosages for a given host can be determined using conventional considerations, e.g., by customary comparison of the differential activities of the subject compositions and of a known agent, e.g., by means of an appropriate, conventional pharmacological protocol.

An important aspect of the present invention is that by performing the modification to the carrier vehicle, the in vivo performance of the bioactive substance can be entirely controlled and predictable. To this extent, while not wishing to be bound to any particular scientific theory, the performance of the bioactive substance is "locked in" immediately post manufacture. As such, the performance of the bioactive substance *in vivo* is comparable (i.e., the active is released to the host such that it remains in the host at a therapeutically effective level for a desired period of time) from the time of manufacture for many months of storage time. As an example, the performance of the bioactive administered *in vivo* 30 days, 60 days, 90 days, 180 days, 360 days or 720 days after manufacture is comparable to the performance just after manufacture. In contrast, in prior formulations when unmodified carrier vehicles are selected, the *in vivo* performance is not as readily predictable as the performance of the bioactive immediately after manufacture may differ from its performance some time after manufacture. The reproducibility, predictability and consistent performance obtained when using the composition of the present invention provides a clear advance in the art.

While the present invention is primarily directed to sustained-release delivery vehicles, it is also expressly contemplated that for certain bioactive substances advantages can be obtained without the vehicle being a sustained-release one. For example, the chemical or physical stability of the final formulation could be improved. Further for parenteral formulations the compositions of this invention may provide reduced injection site irritation for certain tissue irritating bioactive substances. For oral formulations compositions of this invention could possibly provide protection against stomach irritation by certain bioactive substances, may help mask the taste of poorly palatable drugs, and might be used to target delivery of certain drugs (i.e. where it's desired that absorption of the active medicament occur lower in the G.I. tract, rather than in the stomach - for greater efficacy, to target certain disease conditions, etc.) For intramammary formulations compositions of this invention could possibly provide reduced udder irritation, and might prevent or reduce systemic absorption of the drug from the udder, leaving more medicament at the site of the infection, thus improving efficacy and increasing chances for reduced slaughter times.

The invention is further described in the following non-limiting examples.

### Preparation 1- Cottonseed oil modification

A substantially peroxidized unsaturated is prepared from natural cottonseed oil. 105 parts by volume of natural cottonseed oil are added to a vessel having a steam jacket for heating. Steam is applied to the jacket to heat the oil to between about 85 and about 110°C. Air is bubbled through the oil while it is agitated. The flow rate of the air varies from about 1 standard cubic foot per hour (SCFH)/liter to 20 SCFH/liter. Agitation is such that the temperature of the oil remains constant over the time period of heating. The oil is heated for a time and at a temperature necessary to achieve a peroxide value as measured by the method of the US Pharmacopea (USP 24 NF 19 at page 1870) or by AOCS method 8-53 and then cooled, transferred to a different container and stored under nitrogen conditions. To achieve a peroxide value of about 10, at a temperature of about 89°C the oil is heated for about 9 hours, at a temperature of about 100°C the oil is heated for about 3 hours, and at a temperature of about 105°C the oil is heated for about 2.3 hours. To achieve a peroxide value of about 40, at a temperature of about 100°C the oil is heated for about 6.75 hours, and at a temperature of about 105°C the oil is heated for about 5.5 hours. To achieve a peroxide value of about 80, at a temperature of about 105°C the oil is heated for about 8 hours. The relationship between the time and temperature of the oil as compared to its peroxide value is considered to be linear and one skilled in the art could achieve a desired peroxide value depending on the time and temperatures selected for processing.

### Example 1

(i) 10 parts by volume of the modified cottonseed oil prepared according to Preparation 1 and having a peroxide value of between about 10-40 are mixed with 90 parts by volume of Miglyol 812 to form a carrier vehicle.
(ii) 0.1 parts by weight of CCFA are added and mixed for 1-3 hours to form a uniform suspension such that the concentration of CCFA is 100 mg/ml.
(iii) The suspension is heated to about 85 -110°C for about 2-20 hours and permitted to cool.
(iv) The suspension is packaged and sterilized with gamma radiation.

The resulting product is a stable, sustained-release formulation of CCFA having a concentration of 100 mg/ml.

### Example 2

The procedure of Example 1 is repeated except that in step (i) the ratio of modified cottonseed oil to Miglyol 812 is 20:80, and in step (ii) the amount of CCFA added is such that the concentration of CCFA is 200 mg/ml. The resulting product is a sustained-release formulation of CCFA having a concentration of 200 mg/ml.

### Example 3

The procedure of Example 2 is repeated except that steps (iii) and (iv) are replaced with the step of filling vials with the suspension and terminally heating the packaged suspension to 85 -110°C for about 3-14 hours and allowing the vials to cool. The resulting product is a stable, sustained-release formulation of CCFA having a concentration of 200 mg/ml.

### Example 4

The procedure of Example 3 is repeated except that the following step (i) is substituted for the identical step in Example 3 and in step (ii) the amount of CCFA added is such to obtain a final concentration of 100 mg/ml.
(i) Natural peanut oil is modified by heating it to 90-100°C for a period of 1-10 hours to yield a composition having a peroxide value of between 10-80. 1 part of the modified peanut oil by volume is mixed with 99 parts by volume of Miglyol 810 for 1-3 hours.

The resulting product is a stable, sustained-release formulation of CCFA having a concentration of 100 mg/ml.

### Example 5

A formulation is prepared as set forth in Example 1 except that the following step (i) is substituted for the identical step in Example 1, in step (ii) the amount of CCFA added is such to obtain a final concentration of 250 mg/ml, and step (iii) is omitted.
(i) Natural corn oil is modified by heating it to 90-100°C for a period of 1-10 hours to yield a composition having a peroxide value of between of 5-50. 5 parts by volume of this is mixed with 95 parts by volume of Miglyol 829 for 1-3 hours to yield a carrier vehicle.

### Example 6

(i) 40 parts by volume of modified sesame oil prepared using the heat treatment according to Preparation 1 and having a peroxide value of between about 4-60 are mixed with 60 parts by volume of isopropyl myristate to form a carrier vehicle.
(ii) 0.15 parts by weight of ceftiofur crystalline free acid are added and mixed for 1-3 hours to form a uniform suspension such that the concentration of CCFA is 150 mg/ml.
(iii) The suspension is packaged and heated to 85 -110°C for about 3-14 hours and permitted to cool.

The resulting product is a stable, sustained-release formulation of CCFA having a concentration of 150 mg/ml.

### Example 7

(i) 15 parts by volume of safflower oil are mixed with 85 parts by volume of Miglyol 840 to form a carrier vehicle.
(ii) 0.30 parts by weight of CCFA are added and mixed for 1-3 hours to form a uniform suspension such that the concentration of CCFA is 300 mg/ml.
(iii) The suspension is packaged subjected to microwave radiation to modify the safflower oil and permitted to cool.

The resulting product is a stable, sustained-release formulation of CCFA having a concentration of 300 mg/ml.

### Example 8

(i) 100 parts by volume of palm oil are prepared using the heat treatment according to Preparation 1 to have a peroxide value of between about 10-100 to form a carrier vehicle.
(ii) 0.10 parts by weight of ceftiofur crystalline free acid are added and mixed for 1-3 hours to form a uniform suspension such that the concentration of CCFA is 100 mg/ml.
(iii) The suspension is heated to 85 -110°C for about 2-20 hours and permitted to cool.
(iv) The suspension is packaged and sterilized with gamma radiation.

The resulting product is a stable, sustained-release formulation of CCFA having a concentration of 100 mg/ml.

### Testing of Inventive Compositions

Cows suffering from either bovine respiratory disease or an anaerobic infection of the interdigital space are injected with any of the compositions of Examples 1 -8 such that the level of administration of CCFA is between about 4.4 to about 6.6 mg CCFA/kg animal body weight. Administration is by subcutaneous injection in the neck or subcutaneous injection in the ear as described in US 6,074,657. The concentration of effective CCFA metabolites in the blood plasma of the cows rises to at least 0.2 µg/ml within one hour of administration and remains at or above this level for at least 80 to about 140 hours. Only one administration of CCFA is required for the treatment regimen.

Having described the invention in detail and by reference to the preferred embodiments thereof, it will be apparent that modifications and variations are possible without departing from the scope of the appended claims.

## Claims

1. A composition comprising:
(a) crystalline ceftiofur free acid; and
(b) a liquid carrier, said liquid carrier comprising an unsaturated oil having a peroxide value of between 0.1 and 600 milliequivalents (mEq) of peroxide per 1000 grams of oil.

2. The composition according to claim 1 wherein said liquid carrier comprises a mixture of said unsaturated oil with a saturated oil.

3. The composition according to claim 2 wherein said unsaturated oil comprises a substantially peroxidized unsaturated oil having a peroxide value of between 0.1 and 600 milliequivalents (mEq) of peroxide per 1000 grams of oil before it is combined with said saturated oil to form said liquid vehicle.

4. The composition according to claim 1 wherein said liquid carrier comprises a mixture of said unsaturated oil with a non-oxidizing vehicle.

5. The composition according to claims 2 or 4 wherein said unsaturated oil comprises a vegetable oil wherein said vegetable oil is selected from the group consisting of corn oil, peanut oil, sesame oil, olive oil, palm oil, safflower oil, soybean oil, cottonseed oil, rapeseed oil, sunflower oil and mixtures thereof.

6. The composition according to claim 5 wherein said vegetable oil comprises cottonseed oil.

7. The composition according to claim 6 wherein said saturated oil comprises saturated coconut oil.

8. The composition according to claim 7 wherein the ratio by volume of cottonseed oil to saturated coconut oil is between 0.01:99.99 to 30:70.

9. The composition according to claim 8 wherein the ratio by volume of cottonseed oil to saturated coconut oil is between 10:90 to 20:80.

10. The composition according to claim 7 wherein the concentration of crystalline ceftiofur free acid in said composition ranges from 50 mg/ml to 250 mg/ml.

11. The composition according to claim 10 wherein the concentration of crystalline ceftiofur free acid in said composition ranges from 100 mg/ml to 200 mg/ml.

12. A composition consisting essentially of crystalline ceftiofur free acid, cottonseed oil having a peroxide value of between 0.1 and 600 milliequivalents (mEq) of peroxide per 1000 grams of oil and saturated coconut oil wherein the concentration of crystalline ceftiofur free acid in said composition ranges from 100 mg/ml to 200 mg/ml and the ratio by volume of cottonseed oil to saturated coconut oil is between 10:90 to 20:80.

13. A composition according to any one of claims 1 to 12 for the treatment of bacterial infections in mammals.

14. The use of a composition of any one of claims 1 to 12 in the preparation of a medicament for the treatment of bacterial infections in mammals.

## Patentansprüche

1. Zusammensetzung, umfassend:
(a) kristalline freie Säure von Ceftiofur; und
(b) einen flüssigen Träger, wobei der flüssige Träger ein ungesättigtes Öl mit einer Peroxidzahl von zwischen 0,1 und 600 Milliäquivalenten (mÄq) Peroxid pro 1.000 g Öl umfasst.

2. Zusammensetzung gemäß Anspruch 1, wobei der flüssige Träger ein Gemisch aus dem ungesättigten Öl mit einem gesättigten Öl umfasst.

3. Zusammensetzung gemäß Anspruch 2, wobei das ungesättigte Öl ein im Wesentlichen peroxidiertes ungesättigtes Öl mit einer Peroxidzahl von zwischen 0,1 und 600 Milliäquivalenten (mÄq) Peroxid pro 1.000 g Öl umfasst, bevor es mit einem gesättigten Öl kombiniert wird, um das flüssige Vehikel zu bilden.

4. Zusammensetzung gemäß Anspruch 1, wobei der flüssige Träger ein Gemisch aus dem ungesättigten Öl mit einem nicht oxidierenden Vehikel umfasst.

5. Zusammensetzung gemäß den Ansprüchen 2 oder 4, wobei das ungesättigte Öl ein pflanzliches Öl umfasst, wobei das pflanzliche Öl ausgewählt ist aus der Gruppe, bestehend aus Mais- bzw. Getreidekeimöl, Erdnussöl, Sesamöl, Olivenöl, Palmöl, Safloröl, Sojabohnenöl, Baumwollsamenöl, Rapssamenöl, Sonnenblumenöl und Gemischen davon.

6. Zusammensetzung gemäß Anspruch 5, wobei das pflanzliche Öl Baumwollsamenöl umfasst.

7. Zusammensetzung gemäß Anspruch 6, wobei das gesättigte Öl gesättigtes Kokosöl umfasst.

8. Zusammensetzung gemäß Anspruch 7, wobei das Volumenverhältnis von Baumwollsamenöl zu gesättigtem Kokosöl zwischen 0,01:99,99 und 30:70 beträgt.

9. Zusammensetzung gemäß Anspruch 8, wobei das Volumenverhältnis von Baumwollsamenöl zu gesättigtem Kokosöl zwischen 10:90 und 20:80 beträgt.

10. Zusammensetzung gemäß Anspruch 7, wobei die Konzentration an kristalliner freier Säure von Ceftiofur in der Zusammensetzung von 50 mg/ml bis 250 mg/ml reicht.

11. Zusammensetzung gemäß Anspruch 10, wobei die Konzentration an kristalliner freier Säure von Ceftiofur in der Zusammensetzung von 100 mg/ml bis 200 mg/ml reicht.

12. Zusammensetzung, im Wesentlichen bestehend aus kristalliner freier Säure von Ceftiofur, Baumwollsamenöl mit einer Peroxidzahl von zwischen 0,1 und 600 Milliäquivalenten (mÄq) Peroxid pro 1.000 g Öl und gesättigtem Kokosöl, wobei die Konzentration an kristalliner freier Säure von Ceftiofur in der Zusammensetzung von 100 mg/ml bis 200 mg/ml reicht und das Volumenverhältnis von Baumwollsamenöl zu gesättigtem Kokosöl zwischen 10:90 und 20:80 beträgt.

13. Zusammensetzung gemäß einem der Ansprüche 1 bis 12 zur Behandlung von bakteriellen Infektionen bei Säugern.

14. Verwendung einer Zusammensetzung nach einem der Ansprüche 1 bis 12 bei der Herstellung eines Medikaments zur Behandlung bakterieller Infektionen bei Säugern.

## Revendications

1. Composition comprenant :
(a) la forme acide libre, cristalline, du ceftiofur ; et
(b) un support liquide ; ledit support liquide comprenant une huile insaturée ayant un indice de peroxyde compris entre 0,1 et 600 milliéquivalents (mEq) de peroxyde pour 1000 grammes d'huile.

2. Composition selon la revendication 1, dans laquelle ledit support liquide comprend un mélange de ladite huile insaturée avec une huile saturée.

3. Composition selon la revendication 2, dans laquelle ladite huile insaturée comprend une huile insaturée sensiblement peroxydée ayant un indice de peroxyde compris entre 0,1 et 600 milliéquivalents (mEq) de peroxyde pour 1000 grammes d'huile avant qu'elle ne soit combinée avec ladite huile saturée pour former ledit véhicule liquide.

4. Composition selon la revendication 1, dans laquelle ledit support liquide comprend un mélange de ladite huile insaturée avec un véhicule non-oxydant.

5. Composition selon la revendication 2 ou 4, dans laquelle ladite huile insaturée comprend une huile végétale, ladite huile végétale étant sélectionnée dans le groupe consistant en l'huile de maïs, l'huile d'arachide, l'huile de sésame, l'huile d'olive, l'huile de palme, l'huile de carthame, l'huile de soja, l'huile de coton, l'huile de colza, l'huile de tournesol et les mélanges de celles-ci.

6. Composition selon la revendication 5, dans laquelle ladite huile végétale comprend de l'huile de coton.

7. Composition selon la revendication 6, dans laquelle ladite huile saturée comprend de l'huile de coprah saturée.

8. Composition selon la revendication 7, dans laquelle le rapport volumique entre l'huile de coton et l'huile de coprah saturée est compris entre 0,01:99,99 et 30:70.

9. Composition selon la revendication 8, dans laquelle le rapport volumique entre l'huile de coton et l'huile de coprah saturée est compris entre 10:90 et 20:80.

10. Composition selon la revendication 7, dans laquelle la concentration en forme acide libre, cristalline, du ceftiofur, dans ladite composition, se situe entre 50 mg/ml et 250 mg/ml.

11. Composition selon la revendication 10, dans laquelle la concentration en forme acide libre, cristalline, du ceftiofur, dans ladite composition, se situe entre 100 mg/ml et 200 mg/ml.

12. Composition consistant essentiellement en forme acide libre, cristalline, du ceftiofur, en huile de coton ayant un indice de peroxyde compris entre 0,1 et 600 milliéquivalents (mEq) de peroxyde pour 1000 grammes d'huile et en huile de coprah saturée, la concentration en forme acide libre, cristalline, du ceftiofur, dans ladite composition, étant comprise entre 100 mg/ml et 200 mg/ml et le rapport volumique entre l'huile de coton et l'huile de coprah saturée étant compris entre 10:90 et 20:80.

13. Composition selon l'une quelconque des revendications 1 à 12 pour le traitement d'infections bactériennes chez des mammifères.

14. Utilisation d'une composition selon l'une quelconque des revendications 1 à 12 dans la préparation d'un médicament pour le traitement d'infections bactériennes chez des mammifères.
